# EUROPEAN PATENT APPLICATION

(11) **EP 4 445 841 A1**
(43) Date of publication of application: **16.10.2024**
(21) Application number: 22958386.9
(22) Date of filing: 14.09.2022
(51) Int. Cl.: A61B 5/22

(54) **METHOD AND SYSTEM FOR MARKING MOTION DATA AND GENERATING MOTION EVALUATION MODEL**

(71) Applicant: Shenzhen Shokz Co., Ltd., Shenzhen, Guangdong 518108 (CN)
(72) Inventor: LI, Meiqi, Shenzhen, Guangdong 518108 (CN); LIU, Jia, Shenzhen, Guangdong 518108 (CN); SU, Lei, Shenzhen, Guangdong 518108 (CN); ZHOU, Xin, Shenzhen, Guangdong 518108 (CN); LIAO, Fengyun, Shenzhen, Guangdong 518108 (CN); QI, Xin, Shenzhen, Guangdong 518108 (CN)
(74) Representative: Wang, Bo
(86) International application number: PCT/CN2022/118703
(87) International publication number: WO 2024/055192

(57) **Abstract**

Embodiments of the present disclosure provide a method for labeling motion data, comprising: obtaining motion data of a first subject when the first subject is in motion, the motion data representing a motion state of the first subject; obtaining image data of the first subject when the first subject is in motion; and labeling the motion data based on the image data.

## Description

### TECHNICAL FIELD

The present disclosure relates to the field of artificial intelligence technology, and in particular, to a method and system for labeling motion data and generating a motion evaluation model.

### BACKGROUND

With people's increasing attention to physical health and scientific motion, motion monitoring devices are vigorously developing. Devices (such as watches, bracelets, etc.) can recognize simple daily motion behaviors (such as running, walking, simple ball games, etc.). After recognizing the type of motion, these devices can provide simple motion statistical parameters (such as running speed, a count of steps walked, a count of hits, etc.) generally after a long period of time. However, the devices cannot determine whether a user's action or motion mode is correct, especially unable to provide real-time feedback to the user.

Since incorrect motion not only fails to achieve the desired fitness effect but may also cause harm to the human body, it is necessary to provide a motion evaluation method that can recognize users' motion errors in real-time and help users correct them, thereby ensuring the scientific motion of the users.

### SUMMARY

Embodiments of the present disclosure provide a method for labeling motion data, including: obtaining motion data of a first subject when the first subject is in motion, the motion data representing a motion state of the first subject; obtaining image data of the first subject when the first subject is in motion; and obtaining labeled motion data by labeling the motion data based on the image data.

In some embodiments, the labeling the motion data based on the image data includes: sending the image data to a second subject; obtaining labels in labeled image data labeled by the second subject, the labels including a label time and a label content; and labeling the motion data based on the labels.

In some embodiments, the labeling the motion data based on the labels includes: synchronizing the motion data with the image data; and labeling the motion data according to the labels based on the synchronized motion data and the synchronized image data.

In some embodiments, the synchronizing the motion data with the image data includes: determining a labeling action type; determining a first time point corresponding to the labeling action type in the image data; determining a second time point corresponding to the labeling action type in the motion data; and synchronizing the motion data with the image data based on the first time point and the second time point.

In some embodiments, the label time includes an error start time and an error end time.

In some embodiments, the label content includes at least one of: an action type, a target part, or an error type.

In some embodiments, the error type includes at least one of: an injury error, a compensation error, an efficiency error, or a symmetry error.

In some embodiments, the method further comprises modifying the labels based on the motion data.

In some embodiments, the image data includes at least one of: video data, 3D animation data, or model motion pictures of the first subject in motion.

Embodiments of the present disclosure provide a method for generating a motion evaluation model, including: obtaining a training sample set including sets of sample motion data, each set of sample motion data representing a motion state of a first subject; for each set of sample motion data, obtaining labels corresponding to the sample motion data, the labels including a label time and a label content corresponding to the sample motion data; and obtaining the motion evaluation model by training an initial model based on the training sample set and the labels of the sets of sample motion data, the motion evaluation model being configured to evaluate motion data.

In some embodiments, for each set of sample motion data, the obtaining labels corresponding to the sample motion data includes: obtaining sample image data corresponding to the sample motion data; sending the sample image data to a second subject; obtaining labels in labeled sample image data labeled by the second subject; and determining the labels corresponding to the sample motion data based on the labels in the labeled sample image data.

In some embodiments, the determining the labels corresponding to the sample motion data based on the labels in the labeled sample image data includes: synchronizing the sample motion data with the sample image data; and determining the labels corresponding to the sample motion data based on the synchronized sample motion data and the synchronized sample image data.

In some embodiments, the synchronizing the sample motion data with the sample image data includes: determining a labeling action type; determining a first time point corresponding to the labeling action type in the sample image data; determining a second time point corresponding to the labeling action type in the sample motion data; and synchronizing the sample motion data with the sample image data based on the first time point and the second time point.

In some embodiments, the sample image data includes at least one of: sample video data, sample 3D animation data, or sample model motion pictures.

In some embodiments, the label time includes an error start time and an error end time.

In some embodiments, the label content includes at least one of: an action type, a target part, or an error type.

In some embodiments, the error type includes at least one of: an injury error, a compensation error, an efficiency error, or a symmetry error.

In some embodiments, the method further comprises: modifying the labels based on the sample motion data.

In some embodiments, the obtaining labels corresponding to the sample motion data includes: obtaining labeled sample motion data labeled by a second subject; and determining the labels corresponding to the sample motion data based on the labeled motion data.

In some embodiments, the obtaining labels corresponding to the sample motion data further includes: obtaining sample image data corresponding to the sample motion data; and modifying the labels based on the sample image data.

In some embodiments, the obtaining labels corresponding to the sample motion data includes: obtaining sample image data corresponding to the sample motion data; and determining the labels corresponding to the sample motion data based on the sample image data using a labeling model.

In some embodiments, the training an initial model based on the training sample set and the labels of the sets of sample motion data includes one or more iterations, where a current iteration of the one or more iterations includes: for each set of sample motion data, generating a predicted evaluation result using the initial model; determining a loss function value by comparing the predicted evaluation results with the labels of the sets of sample motion data; determining whether the current iteration satisfies a termination condition based on the loss function value; and in response to determining that the current iteration satisfies the termination condition, determining the initial model as the motion evaluation model.

In some embodiments, the method further comprises: updating the motion evaluation model based on feedback data of a third subject.

In some embodiments, the updating the motion evaluation model based on feedback data of a third subject includes: obtaining image data fed back by the third subject; obtaining labels corresponding to the image data fed back by the third subject; obtaining motion data corresponding to the image data fed back by the third subject, the motion data representing a motion state of the third subject; generating a predicted evaluation result based on the motion data using the motion evaluation model; and updating the motion evaluation model based on the predicted evaluation result and the labels corresponding to the image data.

In some embodiments, the method further comprises: displaying the labels corresponding to the image data to the third subject.

In some embodiments, the method further comprises: displaying the motion data corresponding to the image data to the third subject.

Embodiments of the present disclosure provide a system for labeling motion data, including: a first acquisition module, configured to obtain motion data of a first subject when the first subject is in motion, the motion data representing a motion state of the first subject; a second acquisition module, configured to obtain image data of the first subject when the first subject is in motion; and a labeling module, configured to label the motion data based on the image data.

Embodiments of the present disclosure provide a system for generating a motion evaluation model, including: a third acquisition module, configured to obtain a training sample set including sets of sample motion data, each set of sample motion data representing a motion state of a first subject; a fourth acquisition module, configured to for each set of sample motion data, obtain labels corresponding to the sample motion data, the labels including a label time and a label content corresponding to the sample motion data; and a training module, configured to obtain the motion evaluation model by training an initial model based on the training sample set and the labels of the sets of sample motion data, the motion evaluation model being configured to evaluate motion data.

Embodiments of the present disclosure provide a computer-readable storage medium, including executable instructions that, when executed by at least one processor, direct the at least one processor to perform the method for labeling motion data or the method for generating a motion evaluation model described in the present disclosure.

Additional features will be partially elaborated in the following description, and will become apparent to those skilled in the art upon reviewing the following content and accompanying drawings, or can be understood through the creation or operation of examples. The features of the present disclosure can be implemented and obtained through practice or by using various aspects of the methods, tools, and combinations elaborated in the following detailed examples.

### BRIEF DESCRIPTION OF THE DRAWINGS

This description will be further explained in the form of exemplary embodiments, which will be described in detail by means of accompanying drawings. These embodiments are not restrictive, in which the same numbering indicates the same structure, wherein:
FIG. 1 is a schematic diagram illustrating a system for labeling motion data according to some embodiments of the present disclosure;
FIG. 2 is a block diagram illustrating a device for labeling motion data according to some embodiments of the present disclosure;
FIG. 3 is a flowchart illustrating a process for labeling motion data according to some embodiments of the present disclosure;
FIG. 4 is a flowchart illustrating a process for labeling motion data based on labels in labeled image data according to some embodiments of the present disclosure;
FIG. 5 is a schematic diagram illustrating a labeling interface according to some embodiments of the present disclosure;
FIG. 6 is a flowchart illustrating a process for synchronizing motion data and image data according to some embodiments of the present disclosure;
FIG. 7 is a block diagram illustrating a device for generating a motion evaluation model according to some embodiments of the present disclosure;
FIG. 8 is a flowchart illustrating a process for generating a motion evaluation model according to some embodiments of the present disclosure;
FIG. 9 is a flowchart illustrating a process for updating a motion evaluation model according to some embodiments of the present disclosure.

### DETAILED DESCRIPTION

The technical schemes of embodiments of the present disclosure will be more clearly described below, and the accompanying drawings need to be configured in the description of the embodiments will be briefly described below. Obviously, the drawings in the following description are merely some examples or embodiments of the present disclosure, and will be applied to other similar scenarios according to these accompanying drawings without paying creative labor. Unless obviously obtained from the context or the context illustrates otherwise, the same numeral in the drawings refers to the same structure or operation.

As shown in the present disclosure and the patent claims, unless the context clearly suggests exceptions, words such as "a," "an," "one type of," and/or "the" do not specifically refer to the singular and can also include the plural. Generally speaking, the terms "including" and "comprising" only suggest the inclusion of clearly identified steps and elements, and these steps and elements do not constitute an exclusive list. Methods or devices may also contain other steps or elements. The term "based on" means "at least partially based on." The term "one embodiment" represents "at least one embodiment"; the term "another embodiment" represents "at least one additional embodiment."

In the description of the present disclosure, it should be understood that the terms "first," "second," etc. are only used for descriptive purposes and should not be interpreted as indicating or implying relative importance or implicitly specifying the number of indicated technical features. Thus, features qualified by "first," "second," etc. may explicitly or implicitly include at least one such feature. In the description of the present disclosure, "a plurality of" means at least two, such as two, three, etc., unless otherwise specifically and clearly defined.

In the field of sports and fitness, there are over 50 common types of fitness action errors. Even for running, there are over 30 common types of errors. In some embodiments, a motion evaluation model may be used to process user motion data, thereby identifying the error types of the user's actions. However, to accurately identify the error types of the user actions through the model, a large amount of training sample data is required, including user motion data and corresponding labels (e.g., error types). Since the error types are numerous and complex, the motion data and the labels will directly affect the evaluation results of the motion evaluation model.

Therefore, the embodiments of the present disclosure provide a method for labeling motion data and a method for generating a motion evaluation model. The following detailed description, combined with the accompanying drawings, explains the methods for labeling motion data and generating a motion evaluation model provided in the embodiments of the present disclosure.

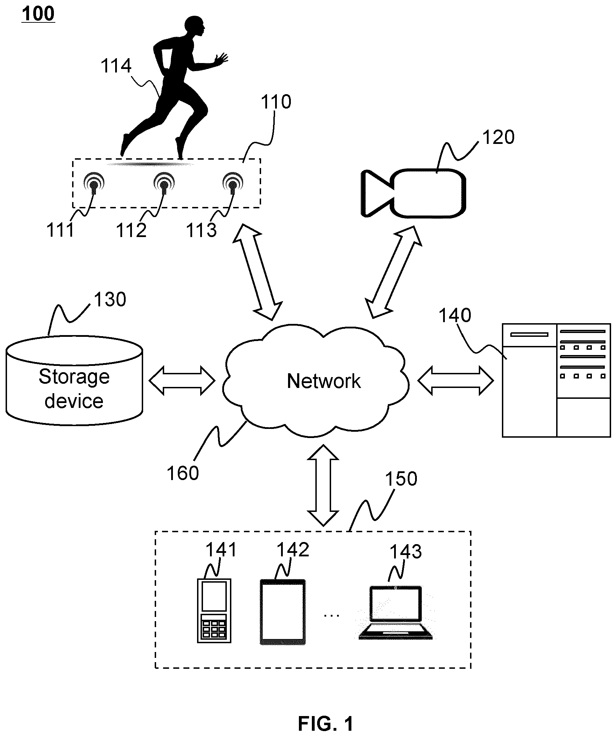

FIG. 1 is a schematic diagram illustrating a system for labeling motion data according to some embodiments of the present disclosure.

According to FIG. 1, in some embodiments, the system 100 for labeling motion data (hereinafter referred to as system 100) may include a motion data acquisition device 110, an image acquisition device 120, a storage device 130, a processing device 140, a terminal device 150, and a network 160. The various components of the system 100 may be connected in a plurality of manners. For example, the motion data acquisition device 110 and/or the image acquisition device 120 may be connected to the storage device 130 and/or the processing device 140 via the network 160, or may be directly connected to the storage device 130 and/or the processing device 140. As another example, the storage device 130 may be directly connected to the processing device 140 or connected via the network 160. As yet another example, the terminal device 150 may be connected to the storage device 130 and/or the processing device 140 via the network 160, or may be directly connected to the storage device 130 and/or the processing device 140.

The motion data acquisition device 110 may obtain motion data from a target subject 114 (also referred to as a first subject). The motion data may refer to a signal generated by the target subject 114 (e.g., a fitness trainee) in motion. Exemplary motion data may include a posture signal, an electromyography signal, a mechanical signal, an electrocardiography signal, a respiratory signal, a sweat signal, etc. In some embodiments, as shown in FIG. 1, the motion data acquisition device 110 may include a posture signal acquisition device 111, an electromyography signal acquisition device 112, and a mechanical signal acquisition device 113. The posture signal acquisition device 111 may include, but is not limited to, a speed sensor, an inertial sensor (e.g., an acceleration sensor, an angular velocity sensor such as a gyroscope), an optical sensor (e.g., an optical distance sensor, a video/an image collector), an acoustic distance sensor, a tension sensor, or any combination thereof. For example, the motion data acquisition device 110 may include a plurality of posture signal acquisition devices 111, which may be placed on different parts of the target subject 114 or have different acquisition angles and/or distances relative to the target subject 114. In some embodiments, the electromyography signal acquisition device 112 may include one or more electrodes. For example, the electromyography signal acquisition device 112 may include a plurality of electrodes that may be used to fit different parts of the target subject 114 (e.g., chest, back, elbows, legs, abdomen, wrists, etc.) to collect electromyography signals from different parts of the target subject 114. In some embodiments, the mechanical signal acquisition device 113 may include a pressure sensor. For example, the pressure sensor may be placed on different parts of the target subject 114 to collect pressure signals from different parts. In some embodiments, the mechanical signals of the target subject may also be determined based on the posture signals and the electromyography signals. In some embodiments, the motion data acquisition device 110 may also include an electrocardiography signal sensor, a respiratory signal sensor, a sweat signal sensor (not shown in FIG. 1), etc. For example, the electrocardiography signal acquisition device may include a plurality of electrodes that may be used to fit different parts of the target subject 114 to collect electrocardiography signals from the target subject 114. As another example, the respiratory signal sensor may include a respiratory rate sensor, a flow sensor, etc., used to detect a respiratory rate, a gas flow, and other data of the target subject 114 in motion. As yet another example, the sweat signal sensor may include a plurality of electrodes that contact the skin of the target subject 114, used to detect a sweat flow of the target subject 114 and analyze sweat components. In some embodiments, the motion data acquisition device 110 may have an independent power source and may send the collected data to other components in the system 100 (e.g., the storage device 130, the processing device 140, the terminal device 150) via wired or wireless connection(e.g., Bluetooth, WiFi, etc.).

The image acquisition device 120 may obtain image data of the target subject 114 in motion. In some embodiments, the image acquisition device 120 may include a camera, a video camera, a monitor, a biometric device, etc., or any combination thereof. In some embodiments, the image acquisition device 120 may continuously or intermittently (periodically or non-periodically) obtain images of the target subject 114 in motion. In some embodiments, the images obtained by the image acquisition device 120 may be matched with the motion data collected by the motion data acquisition device 110 based on the acquisition time. In some embodiments, the image acquisition device 120 may obtain images of the target subject 114 from one or more viewing angles, for example, including one or more of the front, back, left, right, left front, left back, right front, right back, and above viewing angles. In some embodiments, an acquisition frequency of the image acquisition device 120 may be the same as or different from an acquisition frequency of the motion data acquisition device 110.

In some embodiments, the motion data acquisition device 110 and the image acquisition device 120 may send the collected motion data and image data of the target subject 114 to the storage device 130 and the processing device 140, etc., via the network 160. In some embodiments, the processing device 140 may process the motion data collected by the motion data acquisition device 110 and the image data collected by the image acquisition device 120 to label the motion data based on the image data. In some embodiments, the image data may include labels, which may include a label time and a label content (e.g., an error type). The processing device 140 may combine the labels corresponding to the image data to label the motion data collected by the motion data acquisition device 110. In some embodiments, the image acquisition device 120 may send the image data of the target subject 114 to the terminal device 150 via the network 160. In some embodiments, the labels corresponding to the image data may be obtained based on a labeling operation of a second object on the terminal device 150.

The network 160 may facilitate the exchange of information and/or data. The network 160 may include any suitable network that may facilitate the exchange of information and/or data in the system 100. In some embodiments, at least one component of the system 100 (e.g., the motion data acquisition device 110, the image acquisition device 120, the storage device 130, the processing device 140, the terminal device 150) may exchange information and/or data with at least one other component in the system 100 via the network 160. For example, the processing device 140 may obtain the motion data of the target subject 114 in motion from the motion data acquisition device 110 and/or the storage device 130 via the network 160. As another example, the terminal device 150 may obtain the image data of the target subject 114 in motion from the image acquisition device 120 and/or the storage device 130 via the network 160. As yet another example, the processing device 140 may obtain an operation instruction from a second object from the terminal device 150 via the network 160 (e.g., the second object may label the image data collected by the image acquisition device 120 to obtain labels corresponding to the image data).

In some embodiments, the network 160 may be any form of wired or wireless network, or any combination thereof. For example, the network 160 may include a cable network, a wired network, a fiber-optic network, a telecommunications network, an internal network, the Internet, a local area network (LAN), a wide area network (WAN), a wireless local area network (WLAN), a metropolitan area network (MAN), a public switched telephone network (PSTN), a Bluetooth network, a ZigBee network, a near-field communication (NFC) network, etc., or any combination thereof. In some embodiments, the network 160 may include at least one network access point. For example, the network 160 may include wired and/or wireless network access points, such as base stations and/or Internet exchange points. At least one component of the system 100 may connect to the network 160 via an access point to exchange data and/or information.

The storage device 130 may store data, instructions, and/or any other information. In some embodiments, the storage device 130 may store data obtained from the motion data acquisition device 110, the image acquisition device 120, the processing device 140, and/or the terminal device 150. For example, the storage device 130 may store motion data collected by the motion data acquisition device 110 and/or image data collected by the image acquisition device 120. In some embodiments, the storage device 130 may store data and/or instructions used by the processing device 140 to perform or use to complete the exemplary methods described in the present disclosure. In some embodiments, the storage device 130 may include a mass storage, a removable storage, a volatile read-write memory, a read-only memory (ROM), etc., or any combination thereof. Exemplary mass storages may include a magnetic disk, an optical disk, a solid-state disk, etc. In some embodiments, the storage device 130 may be implemented on a cloud platform. For example, the cloud platform may include a private cloud, a public cloud, a hybrid cloud, a community cloud, a distributed cloud, an internal cloud, a multi-layer cloud, etc., or any combination thereof.

In some embodiments, the storage device 130 may be connected to the network 160 to communicate with at least one other component in the system 100 (e.g., the motion data acquisition device 110, the image acquisition device 120, the processing device 140, the terminal device 150). At least one component in the system 100 may access data, instructions, or other information stored in the storage device 130 via the network 160. In some embodiments, the storage device 130 may be directly connected or communicate with one or more components in the system 100 (e.g., the motion data acquisition device 110, the image acquisition device 120, the terminal device 150). In some embodiments, the storage device 130 may be part of the motion data acquisition device 110, the image acquisition device 120, and/or the processing device 140.

The processing device 140 may process data and/or information obtained from the motion data acquisition device 110, the image acquisition device 120, the storage device 130, the terminal device 150, and/or other components of the system 100. In some embodiments, the processing device 140 may obtain motion data of the target subject 114 from any one or more of the motion data acquisition device 110, the storage device 130, or the terminal device 150, and label the motion data based on the image data. In some embodiments, the processing device 140 may also obtain a plurality of sets of sample motion data and labels, and train an initial model based on the plurality of sets of sample motion data and the labels to obtain a motion evaluation model. In some embodiments, the processing device 140 may obtain a pre-stored computer instruction from the storage device 130 and execute the computer instruction to implement the methods described in the present disclosure for labeling motion data and/or model training.

In some embodiments, the processing device 140 may be a single server or a server group. The server group may be centralized or distributed. In some embodiments, the processing device 140 may be local or remote. For example, the processing device 140 may access information and/or data from the motion data acquisition device 110, the image acquisition device 120, the storage device 130, and/or the terminal device 150 via the network 160. As another example, the processing device 140 may be directly connected to the motion data acquisition device 110, the image acquisition device 120, the storage device 130, and/or the terminal device 150 to access information and/or data. In some embodiments, the processing device 140 may be implemented on a cloud platform. For example, the cloud platform may include a private cloud, a public cloud, a hybrid cloud, a community cloud, a distributed cloud, an inter-cloud, a multi-cloud, etc., or any combination thereof.

The terminal device 150 may receive, send, and/or display data. The received data may include data collected by the motion data acquisition device 110, data collected by the image acquisition device 120, data stored in the storage device 130, labeled data of motion data collected by the motion data acquisition device 110 and labeled by the processing device 140, etc. For example, the data received and/or displayed by the terminal device 150 may include motion data collected by the motion data acquisition device 110, the image data collected by the image acquisition device 120, labels labeled by a second object (e.g., a fitness coach) on the image data collected by the image acquisition device 120, labels labeled by the processing device 140 on the motion data collected by the motion data acquisition device 110, etc. The sent data may include input data or instructions from the second object (e.g., labels labeled by a fitness coach on image data collected by the image acquisition device 120).

In some embodiments, the terminal device 150 may include a mobile device 141, a tablet computer 142, a laptop 143, etc., or any combination thereof. For example, the mobile device 141 may include a mobile phone, a personal digital assistant (PDA), a medical mobile terminal, etc., or any combination thereof. In some embodiments, the terminal device 150 may include an input device (such as a keyboard, a touch screen), an output device (such as a display, a speaker), etc. In some embodiments, the processing device 140 may be part of the terminal device 150. In some embodiments, the motion data acquisition device 110 and the image acquisition device 120 may also be part of the terminal device 150. For example, the terminal device 150 may serve as an image acquisition device for collecting image data during the motion of the target subject 114.

It should be noted that the above description of the system 100 is for example and explanation only and does not limit the scope of application of the present disclosure. For those skilled in the art, various modifications and changes may be made to the system 100 under the guidance of the present disclosure. However, these modifications and changes are still within the scope of the present disclosure.

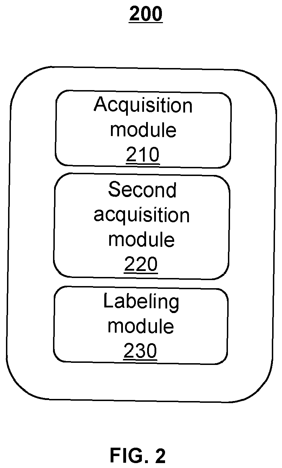

FIG. 2 is a block diagram illustrating a device for labeling motion data according to some embodiments of the present disclosure. In some embodiments, the device 200 for labeling motion data shown in FIG. 2 (hereinafter referred to as "device 200") may be applied to the system 100 shown in FIG. 1 in the form of software and/or hardware. For example, the device 200 may be configured in the processing device 140 and/or the terminal device 150 in the form of software and/or hardware to label the motion data collected by the motion data acquisition device 110.

According to FIG. 2, in some embodiments, the device 200 may include a first acquisition module 210, a second acquisition module 220, and a labeling module 230.

The first acquisition module 210 may be configured to obtain motion data of the target subject 114. For example, the first acquisition module 210 may obtain motion data from any one or more of the motion data acquisition device 110, the storage device 130, or the terminal device 150. In some embodiments, the motion data may be used to characterize a motion state of the subject, and may include a posture signal, an electromyography signal, a mechanical signal, an electrocardiography signal, a respiratory signal, a sweat signal, etc., or any combination thereof. The posture signal may include information such as an angle, a speed, and an acceleration of each joint, or an Euler angle, an angular velocity, and an angular acceleration of various parts of the human body. The electromyography signal refers to a physiological signal collected from the human body through electrodes or other electromyography acquisition devices, which reflects the electromyography situation of the human body. The mechanical signal may refer to the force exerted on the joints of the target subject 114 or the force detected by motion equipment. In some embodiments, the mechanical signal may be obtained through a mechanical sensor. For example, the mechanical sensor may include a pressure sensor, and pressure signals from different parts of the target subject 114 may be obtained based on the pressure sensor as the mechanical signal of the target subject 114. In some embodiments, the mechanical signal may be determined based on the posture signal and the electromyography signal. The electrocardiography signal refers to a signal that indicates the heart activity of the target subject 114. In some embodiments, the electrocardiography signal may be collected through an electrocardiography signal acquisition device. The respiratory signal refers to a signal that indicates the respiratory state of the target subject 114. In some embodiments, the respiratory signal may be collected through a respiratory signal acquisition device. For example, the respiratory signal acquisition device may include a respiratory rate sensor, a flow sensor, etc., which are used to detect a respiratory rate, a gas flow rate, and other data of the target subject 114 in motion. The sweat signal refers to a signal that indicates the sweating condition of the target subject 114. In some embodiments, the sweat signal may be collected through a sweat signal acquisition device. For example, the sweat signal acquisition device may include a plurality of electrodes that contact the skin of the target subject 114, which are used to detect a sweat flow rate of the target subject 114 or analyze sweat composition.

The second acquisition module 220 may be configured to obtain image data of the target subject 114 when the target subject is in motion. In some embodiments, the image data may be images or videos collected by the image acquisition device 120 during the motion of the target subject 114. For example, the image data may be videos continuously collected by the image acquisition device 120 during the motion of the target subject 114. As another example, the image data may be images intermittently (e.g., periodically or non-periodically) collected by the image acquisition device 120 during the motion of the target subject 114. In some embodiments, the image data may be collected from one or a plurality of viewing angles. For example, exemplary viewing angles may include one or more of the front, back, left, right, left front, left back, right front, right back, and above. In some embodiments, the image data may also include 3D animation data or model motion pictures. The 3D animation data or model motion pictures may be reconstructed based on images or videos collected from a plurality of viewing angles, or may be calculated based on sensor data collected by one or more sensors connected or attached to the target subject 114.

The labeling module 230 may be configured to label the motion data obtained by the first acquisition module 210 based on the image data obtained by the second acquisition module 220. For example, in some embodiments, the image data may be labeled to obtain labels of the image data. The labels may include a label time and a label content (e.g., error types of a motion and error times reflected in the image data of the target subject). The labeling module 230 may match the motion data based on the label time in the image data and then label the motion data based on the label content.

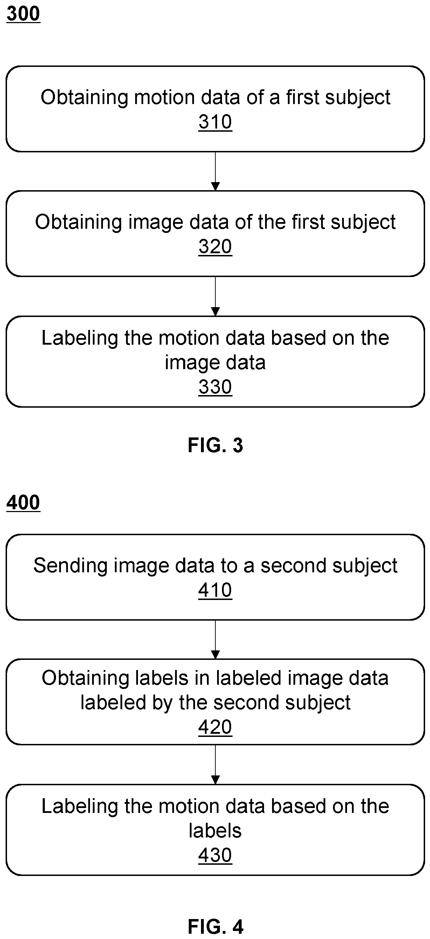

More descriptions regarding each module mentioned above may be found elsewhere in the present disclosure (e.g., the sections related to FIGs. 3-6), which is not repeated here.

It should be understood that the device 200 and modules shown in FIG. 2 may be implemented in various manners. For example, in some embodiments, the system and modules may be implemented through hardware, software, or a combination of software and hardware. The hardware part may be implemented using dedicated logic; the software part may be stored in memory and executed by an appropriate instruction execution system, such as a microprocessor or specially designed hardware. Those skilled in the art can understand that the above methods and systems can be implemented using computer-executable instructions and/or code contained in processor control codes, such as codes provided on carrier media like disks, CDs, or DVD-ROMs, programmable memory such as read-only memory (firmware), or data carriers such as optical or electronic signal carriers. The system and modules described in the present disclosure may be implemented not only by hardware circuits such as very large-scale integrated circuits or gate arrays, semiconductors such as logic chips or transistors, or programmable hardware devices such as field-programmable gate arrays or programmable logic devices, but also by software executed by various types of processors, or by a combination of the above hardware circuits and software (e.g., firmware).

It should be noted that the description of the device 200 is provided for illustrative purposes only and is not intended to limit the scope of the present disclosure. It can be understood that for those skilled in the art, various modules can be arbitrarily combined or connected to other modules as subsystems based on the description of the present disclosure, without departing from this principle. For example, the first acquisition module 210, the second acquisition module 220, and the labeling module 230 disclosed in FIG. 2 may be different modules in a system, or a single module that performs the functions of two or more of the above modules. For example, the first acquisition module 210 and the second acquisition module 220 may be two separate modules, or a single module with different data acquisition functions. As another example, the device 200 may also include a label modification module that may modify the labels of the image data based on the motion data. As another example, each module may share a storage module, or each module may have its own storage module. Such variations are within the scope of the present disclosure.

FIG. 3 is a flowchart illustrating a process for labeling motion data according to some embodiments of the present disclosure. In some embodiments, process 300 may be executed through processing logic, which may include hardware (e.g., circuitry, dedicated logic, programmable logic, microcode, etc.), software (instructions running on a processing device to perform hardware simulation), etc., or any combination thereof. In some embodiments, one or more operations in process 300 illustrated in FIG. 3 may be implemented by the processing device 140 and/or the terminal device 150 shown in FIG. 1. For example, process 300 may be stored in the form of instructions in the storage device 130, and invoked and/or executed by the processing device 140 and/or the terminal device 150. The following descriptions take the execution of process 300 performed by the processing device 140 as an example.

According to FIG. 3, in some embodiments, the process 300 for labeling motion data may include the following operations.

Operation310, motion data of a first subject may be obtained. In some embodiments, operation 310 may be executed by the first acquisition module 210.

In some embodiments, the motion data refers to a signal generated by a target subject (also referred to as the first subject) in motion. The motion data may be used to characterize a motion state of the target subject. Exemplary motion data may include any one or a combination of an electromyography signal, a posture signal, a mechanical signal, an electrocardiography signal, a respiratory signal, and a sweat signal. In some embodiments, the electromyography signal may characterize the technical accuracy (e.g., a muscle recruitment order) and an injury risk (e.g., a fatigue level) of the target subject's current motion. In some embodiments, the electromyography signal may be collected through one or more electrodes attached to the target subject. For example, the plurality of electrodes may be attached to different parts of the target subject (e.g., chest, back, elbow, leg, abdomen, wrist, etc.) to collect electromyography signals from different parts of the target subject.

The posture signal may include information such as a joint angle, a speed, an acceleration of each joint, or an Euler angle, an angular velocity, an angular acceleration, etc. of various body parts. In some embodiments, the posture signal may also be used to characterize the technical accuracy (e.g., a joint angle, a force generation sequence, etc.) and an injury risk (e.g., a shoulder impingement) of the target subject's current motion. In some embodiments, the posture signal may be collected by a posture signal acquisition device (e.g., the posture signal acquisition device 111 shown in FIG. 1). Exemplary posture signal acquisition devices may include a speed sensor, an inertial sensor (e.g., an acceleration sensor, an angular velocity sensor (such as a gyroscope), etc.), an optical sensor (e.g., an optical distance sensor, a video/an image collector), an acoustic distance sensor, etc., or any combination thereof.

The mechanical signal refers to a force exerted on a joint of the target subject or detected by motion equipment, which may characterize an injury risk (e.g., an ankle pressure, a knee pressure, etc.). In some embodiments, the mechanical signal may be obtained through a mechanical sensor. For example, the mechanical sensor may include a pressure sensor that obtains pressure signals of different parts of the target subject as the mechanical signals of the target subject. In some embodiments, the mechanical signal may be determined based on the posture signal and the electromyography signal.

The electrocardiography signal refers to a signal representing the heart activity of the target subject. In some embodiments, the electrocardiography signal may be collected by an electrocardiography signal acquisition device. For example, the electrocardiography signal acquisition device may include a plurality of electrodes that may be attached to different parts of the target subject to collect electrocardiography signals from the target subject. The respiratory signal refers to a signal representing a respiratory state of the target subject. In some embodiments, the respiratory signal may be collected by a respiratory signal acquisition device. For example, the respiratory signal acquisition device may include a respiratory frequency sensor and a flow sensor for detecting a respiratory frequency, a gas flow rate, and other data of the target subject in motion. The sweat signal refers to a signal representing the sweating condition of the target subject. In some embodiments, the sweat signal may be collected by a sweat signal acquisition device. For example, the sweat signal acquisition device may include a plurality of electrodes in contact with the skin of the target subject to detect a sweat flow rate or analyze the sweat components of the target subject.

In some embodiments, the processing device 140 may directly obtain the motion data from a motion data acquisition device (e.g., the motion data acquisition device 110). In some embodiments, the motion data may be stored in a storage device (e.g., the storage device 130), and the processing device 140 may obtain the motion data from the storage device.

Operation 320, image data of the first subject may be obtained. In some embodiments, operation 320 may be performed by the second acquisition module 220.

In some embodiments, the processing device 140 may obtain image data of a target subject in motion. In some embodiments, the processing device 140 may directly obtain the image data from an image acquisition device (e.g., the image acquisition device 120). In some embodiments, the image data may be collected by an image acquisition device and stored in the storage device 130, and the processing device 140 may obtain the image data from the storage device 130. In some embodiments, the image data may include any one of video data, 3D animation data, or model motion pictures of the target subject in motion. For example, the processing device 140 may obtain videos of the target subject captured by the image acquisition device from multiple viewing angles, and directly use videos as the image data of the target subject, or reconstruct 3D animation data or model motion pictures based on the videos. As another example, the processing device 140 may obtain sensor data collected by one or more sensors (e.g., an inertial sensor, an attitude sensor, an ultrasonic sensor, etc.) connected or attached to the target subject, and determine 3D animation data or model motion pictures based on the sensor data.

In some embodiments, the image data may be matched with the motion data based on an acquisition time or a data feature. For example, when the motion data collected by the motion data acquisition device 110 and the image data collected by the image acquisition device 120 have the same acquisition time, which indicates that the motion data and the image data correspond to the same motion process of the target subject, the motion data and the image data may be matched or associated. As another example, when a certain data point (or segment) in the image data corresponds to the same action as a certain data point (or segment) in the motion data, the motion data and the image data may be matched or associated.

Operation 330, the motion data may be labeled based on the image data. In some embodiments, operation 330 may be performed by the labeling module 230.

In some embodiments, the processing device 140 may label the motion data based on the image data. In some embodiments, the processing device 140 may first send the image data to a second subject (e.g., a fitness coach), for example, by transmitting the image data to the second subject's terminal device 150. After receiving the image data, the second subject may determine whether there are errors in the actions of the target subject in the image data. If there are errors, labels of the image data may be input via the terminal device 150. Furthermore, the processing device 140 may obtain labeled image data from the second subject, extract the labels from the labeled image data, and then use the labels to label the motion data. More descriptions regarding labeling the motion data based on the image data may be found elsewhere in the present disclosure (e.g., FIGs. 4-6 and relevant descriptions thereof), which is not repeated here.

In some embodiments, the labeled motion data and labeled image data may be stored in the storage device 130. For example, the labeled motion data and labeled image data may be stored on a cloud platform, and the second subject may access and view the labeled motion data and/or the labeled image data from the cloud platform via the terminal device 150 and the network 160.

In some embodiments, the processing device 140 or the second subject may also modify the labels in the labeled image data. For example, if the second subject finds errors in the labels when reviewing the labeled motion data and/or the labeled image data, modified labels may be input via the terminal device 150 to replace the original labels. In some embodiments, the labels of the image data may be modified based on the motion data. For example, after one second subject labels the image data, the processing device 140 may send the labeled image data and the corresponding motion data to another second subject. The other second subject may view the labeled image data and the corresponding motion data via a terminal device. Merely by way of example, the labeled image data and the corresponding motion data may be displayed for comparison on the other second subject's terminal device. Based on the displayed motion data, the other second subject may determine whether the labels in the labeled image data provided by the second subject are accurate. If the labels in the labeled image data are inaccurate, the other second subject may modify the labels. Furthermore, the processing device 140 may obtain the modified labels from the other second subject as the labels of the image data. The labels of the image data may also serve as the labels of the motion data.

It should be noted that the above description related to process 300 is for illustrative and explanatory purposes only and does not limit the scope of application of the present disclosure. Those skilled in the art can make various amendments and modifications to process 300 under the guidance of the present disclosure. However, these amendments and modifications remain within the scope of the present disclosure.

FIG. 4 is a flowchart illustrating a process for labeling motion data based on labels in labeled image data according to some embodiments of the present disclosure. In some embodiments, process 400 may be executed through processing logic, which may include hardware (e.g., circuitry, dedicated logic, programmable logic, microcode, etc.), software (instructions executed on a processing device to perform hardware simulation), etc., or any combination thereof. In some embodiments, one or more operations in process 400 illustrated in FIG. 4 may be implemented by the processing device 140 and/or the terminal device 150 depicted in FIG. 1. For example, process 400 may be stored in the form of instructions in the storage device 130 and invoked and/or executed by the processing device 140 and/or the terminal device 150. In some embodiments, operation 330 of process 300 may be implemented through process 400. In some embodiments, process 400 may be executed by the labeling module 230. The following descriptions take the execution of process 400 performed by the processing device 140 as an example.

According to FIG. 4, in some embodiments, process 400 may include the following operations.

Operation 410, the image data may be sent to a second subject.

In some embodiments, the processing device 140 may send the image data to the second subject. The second subject may refer to a user capable of labeling the image data. For example, the processing device 140 may transmit the image data stored in the storage device to the terminal device (e.g., the terminal device 150) of the second subject. As another example, the processing device 140 may send the image data collected by a motion data acquisition device (e.g., the motion data acquisition device 110) in real time to the terminal device of the second subject. It should be noted that the first subject (or target subject) and the second subject described in the present disclosure may be different parties performing different operations, or may be the same subject performing different operations. For example, the first subject may be a fitness trainee, and the second subject may be a fitness coach labeling the fitness trainee's image data. As another example, both the first subject and the second subject may be a fitness coach who may label his image data.

Operation 420, labels in labeled image data labeled by the second subject may be obtained.

In some embodiments, the second subject may view the image data on a terminal device and label the image data. For example, the second subject may view the image data through an application on the terminal device. The application's interface may include input components such as a text input box, a drop-down menu, a selection button, etc. The second subject may input and/or select labels for the image data using these input components, thereby labeling the image data. Furthermore, the processing device 140 may obtain the labels from the labeled image data.

In some embodiments, the labels in the labeled image data may include a label time and a label content. The label time may include an action error time of the target subject, such as an error start time and an error end time. The label content may include at least one of an action type, a target part, and an error type of the target subject. In some embodiments, the target part refers to the part of the target subject where the action error occurs. The error type may include at least one of an injury error, a compensation error, an efficiency error, a symmetry error, etc. The injury error refers to a motion error that may cause harm to the human body. The compensation error may refer to an error in which a non-target part (e.g., a muscle) is used to assist in exerting force. The efficiency error may refer to that a range of action is too large or too small when the action is performed in a certain mode such that the target part is in a non-optimal activation state. The symmetry may refer to an imbalance in force generation between two symmetrical (e.g., bilaterally symmetrical, anteroposteriorly symmetrical) parts of the body. In some embodiments, the error type may also include a specific error content. For example, the specific error content of injury errors varies across different action types. Merely by way of example, in a lat pulldown action, the target part may include the upper arm, and a specific error content of an injury error may be that an internal rotation angle exceeds a preset angle threshold. In some embodiments, label content related to the error type may include both the error type and the specific error content. In some embodiments" the specific error content may replace the error type in the label content. For example, the label content may be "injury error - internal rotation angle exceeding the preset angle threshold" or simply "internal rotation angle exceeding the preset angle threshold." In some embodiments, the second subject may label correct actions in the image data to generate labels corresponding to the correct actions. Correspondingly, the label time may include a correct action time of the target subject, such as th start time and the end time of the correct action. The label content may include at least one of an action type, a target part, and an identifier of the correct action (e.g., text or symbols indicating a correct action).

In some embodiments, the label content may be arranged according to a preset rule. For example, the action type, the target part, and the error type may serve as primary, secondary, and tertiary labels, respectively, arranged in that order within the label content. In some embodiments, one or more of the action type, the target part, and the error type in the label content may be interchangeable or combined arbitrarily. For example, the action type may include the target part and/or exercise equipment used in motion such that the target part and/or exercise equipment used in motion may replace the action type in the label content.

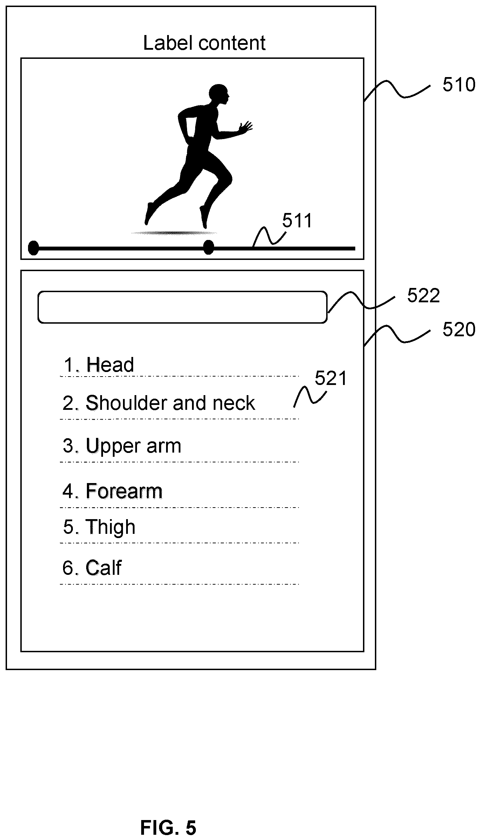

Merely by way of example, FIG. 5 is a schematic diagram illustrating a labeling interface according to some embodiments of the present disclosure. As shown in FIG. 5, the application interface on the second subject's terminal device may include an image data display component 510 and an input component 520. The image data display component 510 is used to display image data (e.g., video data), and the input component 520 is used to input labels of the image data. For example, below the image data, there is a progress bar 511 aligned with the playback time of the image data. The second subject may select the start time and end time of the error by manipulating (e.g., clicking or sliding) the progress bar 511 and input the label content using the input component 520. As shown in FIG. 5, the input component 520 may include a drop-down menu 521, and the second subject may select the label content through the drop-down menu 521. In some embodiments, the drop-down menu may include multiple levels corresponding to label content, such as a primary menu for action types, a secondary menu for target parts, and a tertiary menu for error types. The second subject may make selections from each level of the drop-down menu. In some embodiments, the second subject may also customize the label content. As another example, the input component 520 may include an input box 522. The second subject may enter keyword(s) in the input box 522 to search for related labels in the drop-down menu or directly input the label content. In some embodiments, the label content input by the second subject may be displayed on the interface. As shown in FIG. 5, the label content input by the second subject may be displayed at the top of the interface. It should be noted that the labeling interface shown in FIG. 5 is for illustrative and explanatory purposes only and does not limit the scope of the present disclosure. In some embodiments, the image data may be labeled using other methods. For example, an action recognition or motion evaluation program/model may process the image data to generate recommended information for label time and/or label content, which is then displayed to the second subject for selection or modification. As another example, the second subject may input label time and/or label content via voice or electroencephalogram.

Operation 430, the motion data may be labeled based on the labels.

In some embodiments, the processing device 140 may obtain the labels from the labeled image data and label the motion data based on the labels. In some embodiments, to avoid mismatches between the labels in the image data and the motion data due to desynchronization between the motion data and the image data, the processing device 140 may synchronize the motion data with the image data. Further, based on the synchronized motion data and synchronized image data, the processing device 140 may label the motion data using the labels. For example, the processing device 140 may use the labels of the image data as labels of the motion data, where the label time in the motion data may be determined based on the label time in the image data, and the label content of the image data may be used as the label content of the motion data at the label time.

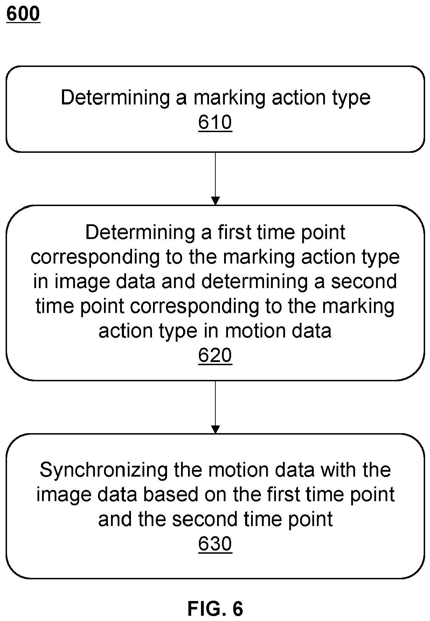

In some embodiments, both the motion data and the image data may include corresponding acquisition timestamps, and the processing device 140 may synchronize the motion data and image data based on these timestamps. In some embodiments, to avoid synchronization errors due to desynchronization between the system clocks of the motion data acquisition device 110 and the image acquisition device 120, the processing device 140 may synchronize the motion data and the image data based on marking action types. In some embodiments, the processing device 140 may also synchronize the motion data and the image data based on hardware information related to the motion data acquisition device 110 and the image acquisition device 120. More descriptions regarding synchronizing the motion data and the image data may be found elsewhere in the present disclosure (e.g., FIG. 6 and relevant descriptions thereof), which is not repeated here.

It should be noted that the above description related to process 400 is merely for illustration and explanation, and does not limit the scope of application of the present disclosure. For those skilled in the art, various modifications and changes can be made to Process 400 under the guidance of the present disclosure. However, these modifications and changes are still within the scope of the present disclosure.

FIG. 6 is a flowchart illustrating a process for synchronizing motion data and image data according to some embodiments of the present disclosure. In some embodiments, process 600 may be executed through processing logic, which may include hardware (e.g., circuitry, dedicated logic, programmable logic, microcode, etc.), software (instructions running on a processing device to perform hardware simulation), etc., or any combination thereof. In some embodiments, one or more operations in process 600 illustrated in FIG. 6 may be implemented by the processing device 140 and/or the terminal device 150 shown in FIG. 1. For example, process 600 may be stored in the form of instructions in the storage device 130 and invoked and/or executed by the processing device 140 and/or the terminal device 150. In some embodiments, at least a portion of operation 430 in process 400 may be implemented through process 600. In some embodiments, process 600 may be executed by the labeling module 230. The following descriptions take the execution of process 600 performed by the processing device 140 as an example.

According to FIG. 6, in some embodiments, process 600 may include the following operations.:
Operation 610, a marking action type may be determined.

In some embodiments, to synchronize the motion data and the image data, the processing device 140 may determine a marking action type. The marking action type may refer to a starting action when the target subject begins a motion. For example, the starting action may be an action with a significant motion change compared to a previous motion. As another example, the starting action may be a specific action. In some embodiments, for ease of recognition, the marking action may be unrelated to a motion type of the target subject. For example, when the motion type of the target subject is running, the marking action may be clapping, jumping, arm spreading, etc.

Operation 620, a first time point corresponding to the marking action type may be determined in the image data, and a second time point corresponding to the marking action type may be determined in the motion data.

In some embodiments, the processing device 140 may process the image data and the motion data, and determine the first time point corresponding to the marking action type in the image data and the second time point corresponding to the marking action type in the motion data. The first and second time points may refer to the same moment of the marking action in the image data and motion data, respectively. In some embodiments, the first and second time points may represent any time point (e.g., a starting time point, a midpoint, an ending time point, etc.) within the time period corresponding to the marking action in the image data and the motion data.

Operation 630, the motion data may be synchronized with the image data based on the first time point and the second time point.

In some embodiments, the processing device 140 may synchronize the image data with the motion data based on the first time point and the second time point. For example, the first time point and the second time point may serve as identifiers for the same moment in the image data and the motion data. The processing device 140 may align the first time point in the image data with the second time point in the motion data, thereby synchronizing the image data with the motion data.

It should be noted that the above description related to process 600 is merely for illustration and explanation, and does not limit the scope of application of the present disclosure. Those skilled in the art can make various modifications and changes to process 600 under the guidance of the present disclosure. However, these modifications and changes remain within the scope of the present disclosure. In some embodiments, the motion data and the image data may include corresponding acquisition timestamps, and the processing device 140 may synchronize the motion data with the image data based on the timestamps. In some embodiments, the processing device 140 may synchronize the motion data with the image data based on hardware information related to the motion data acquisition device 110 and the image acquisition device 120. For example, the motion data acquisition device 110 and the image acquisition device 120 may be an integrated data acquisition system. The processing device 140 may control the data acquisition system to synchronously obtain the image data and the motion data based on the hardware information related to the motion data acquisition device 110 and the image acquisition device 120, thereby achieving the synchronization of the image data and the motion data.

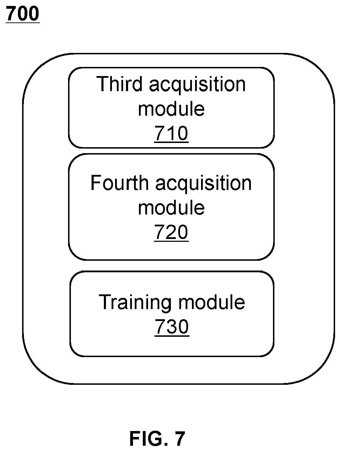

In some embodiments, after labeling the motion data using the above methods, the labeled motion data may be used as training samples. Based on the training samples, a motion evaluation model may be trained to generate a motion evaluation model for evaluating motion data. The following provides a detailed description of the device and method for generating a motion evaluation model provided in the embodiments of the present disclosure, in combination with FIGs. 7-9.

FIG. 7 is a block diagram illustrating a device for generating a motion evaluation model according to some embodiments of the present disclosure.

In some embodiments, the device 700 for generating a motion evaluation model shown in FIG. 7 (also referred to as device 700) may be applied to system 100 shown in FIG. 1 in the form of software and/or hardware. For example, device 700 may be configured in the processing device 140 and/or the terminal device 150 in software and/or hardware form to generate a motion evaluation model. According to FIG. 7, in some embodiments, the device 700 may include a third acquisition module 710, a fourth acquisition module 720, and a training module 730.

The third acquisition module 710 may be configured to obtain a training sample set including a plurality of sets of sample motion data. Each set of sample motion data may include sample data representing a motion state of a first subject. For example, each set of sample motion data may include a sample electromyography signal, a sample posture signal, a sample mechanical signal, a sample electrocardiography signal, a sample respiratory signal, a sample sweat signal, etc., or any combination thereof.

The fourth acquisition module 720 may be configured to, for each set of sample motion data, obtain labels corresponding to the sample motion data. The labels may include a label time and a label content corresponding to the sample motion data. In some embodiments, the labels corresponding to the sample motion data may be obtained according to the process of labeling motion data illustrated in FIG. 3.

The training module 730 may be configured to train an initial model based on the training sample set and labels to obtain a motion evaluation model. The motion evaluation model may be configured to evaluate motion data. In some embodiments, the motion evaluation model may be a machine learning model that, after being trained using the aforementioned sample motion data and the labels, can evaluate motion data and identify error types of the subject.

The above description of device 700 is provided for illustrative purposes only and is not intended to limit the scope of the present disclosure. It can be understood that those skilled in the art can, based on the description of the present disclosure, make any combination of various modules or form subsystems connected to other modules without departing from this principle. For example, the third acquisition module 710, the fourth acquisition module 720, and the training module 730 in FIG. 7 may be different modules in a system, or a single module that may implement the functions of two or more of the above modules. For example, the third acquisition module 710 and the fourth acquisition module 720 may be two separate modules, or a single module with different data acquisition functions. As another example, device 200 and device 700 may be different devices or a device with a combination of modules with different functions. Furthermore, device 700 may also include a label modification module that may modify the labels of the sample image data based on sample motion data. Such variations are all within the scope of protection of the present disclosure.

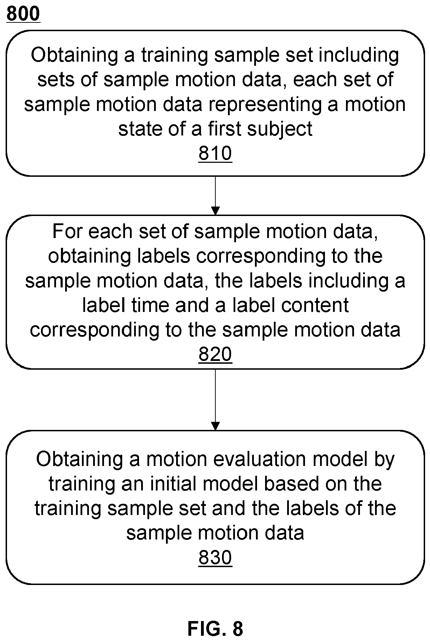

FIG. 8 is a flowchart illustrating a process for generating a motion evaluation model according to some embodiments of the present disclosure. In some embodiments, process 800 may be executed through processing logic, which may include hardware (e.g., circuits, dedicated logic, programmable logic, microcode, etc.), software (instructions running on a processing device to perform hardware simulation), etc., or any combination thereof. In some embodiments, one or more operations in process 800 may be implemented through the processing device 140 and/or the terminal device 150 shown in FIG. 1. In some embodiments, process 800 shown in FIG. 8 may be executed by a separate model training device. The following descriptions take the execution of process 800 performed by the processing device 140 as an example.

According to FIG. 8, in some embodiments, process 800 may include the following operations.

Operation 810, a training sample set including sets of sample motion data may be obtained, each set of sample motion data representing a motion state of a first subject. In some embodiments, operation 810 may be executed by the third acquisition module 710.

In some embodiments, the sample motion data may be collected by a motion data acquisition device (e.g., the motion data acquisition device 110 in FIG. 1). For example, motion data from multiple sample subjects may be collected using the motion data acquisition device to generate the sample motion data. In some embodiments, the processing device 140 may obtain multiple sets of sample motion data from the motion data acquisition device and/or the storage device (e.g., the storage device 130) to obtain the training sample set. In some embodiments, each set of sample motion data in the training sample set may include a sample electromyography signal, a sample posture signal, a sample mechanical signal, a sample electrocardiography signal, a sample respiratory signal, a sample sweat signal, etc., or any combination thereof.

Operation 820, for each set of sample motion data, labels corresponding to the sample motion data may be obtained, the labels including a label time and a label content corresponding to the sample motion data. In some embodiments, operation 820 may be executed by the fourth acquisition module 720.

In some embodiments, the processing device 140 may obtain labels corresponding to the sample motion data. For example, the processing device 140 may obtain sample image data corresponding to the sample motion data, send the sample image data to a second subject, and obtain the labels of labeled sample image data labeled by the second subject. Further, based on the labels in the labeled sample image data, the labels corresponding to the sample motion data may be determined. The sample image data corresponding to the sample motion data refers to the image data collected during the motion process of the target subject generating the sample motion data. For example, when the sample motion data is collected from the beginning to the end of the running process of a target subject, the sample image data may be the image data collected during the same running process.

In some embodiments, the sample image data may be collected by an image acquisition device (e.g., the image acquisition device 120 in FIG. 1) and stored in the storage device 130. In some embodiments, the sample image data may include video data, 3D animation data, or model motion pictures of the target subject in motion. After the image acquisition device collects the sample image data, the sample image data may be sent to the second subject (e.g., to the terminal device 150). The second subject may determine whether there are any errors in the target subject's motions in the sample image data. If there are errors, the second subject may input the labels for the sample image data via the terminal device. Further, the processing device 140 may obtain the labels of the labeled sample image data and determine the labels corresponding to the sample motion data based on the labels of the labeled sample image data.

In some embodiments, the labels inputted by the second subject for the sample image data may include a label time and a label content. The label time may include an error start time and an error end time, while the label content may include at least one of an action type, a target part, and an error type. The error type includes at least one of an injury error, a compensation error, an efficiency error, a symmetry error, etc.

In some embodiments, the processing device 140 may label the sample image data using a labeling model and determine the labels corresponding to the sample motion data based on the labeled sample image data. The labeling model may be a machine learning model. When the labeling model is trained, image data may be labeled to determine labels of the image data. The labels include a label time and a label content. Furthermore, the labeled image data may be used as input for training the machine learning model to obtain a marking model. The marking model may output labels when sample image data is inputted.

In some embodiments, to determine the labels corresponding to the sample motion data based on the labeled sample image data, the processing device 140 may synchronize the sample motion data with the sample image data. For example, the processing device 140 may identify a marking action type, determine a first time point corresponding to the marking action type in the sample image data and a second time point corresponding to the marking action type in the sample motion data, and synchronize the sample motion data with the sample image data based on the first time point and the second time point.

In some embodiments, the labeled sample motion data and the labeled sample image data may be stored in the storage device 130. For example, the labeled sample motion data and the labeled sample image data may be stored on a cloud platform, and the second subject may access and view the labeled data via the terminal device 150 and the network 160.

In some embodiments, the processing device 140 or the second subject may modify the labels. For example, if the second subject finds errors in the labels when viewing the labeled motion data or the labeled image data, the second subject may input modified labels via the terminal device 150 to replace the original labels.

In some embodiments, operation 820 may be the same or similar to process 300. More descriptions regarding determining the labels corresponding to the sample motion data based on the sample image data may be found elsewhere in the present disclosure (e.g., FIGs. 3-6 and relevant descriptions thereof).

In some embodiments, the labels may be directly determined based on the sample motion data. For example, the processing device 140 may send the sample motion data to the terminal device 150, and the second subject may view and directly label the sample motion data. Merely by way of example, when there is a specific error type, the sample motion data of the target part may be presented in a specific manner. The second object may determine that the label corresponding to the sample motion data is the specific error type when the sample motion data is presented in the specific manner. In some embodiments, the processing device 140 may obtain the labeled sample motion data labeled by the second object, thereby determining the label corresponding to the sample motion data.

In some embodiments, it may be difficult for the second subject to intuitively find errors in the motion of the target subject when labeling the sample motion data, such that it may not be accurate to determine the label corresponding to the sample motion data based on the labels of the labeled sample motion data labeled by the second object. To improve accuracy, in some embodiments, the processing device 140 may modify the labels based on the sample image data corresponding to the sample motion data. For example, the processing device 140 may send the labeled sample motion data and the corresponding sample image data to the second subject, and the second object may view the label of the labeled sample motion data through the terminal device and determine whether the labels are accurate in combination with the corresponding sample image data. If the labels are inaccurate, the second object may modify the labels of the labeled sample motion data. Further, the processing device 140 may obtain the labels modified by the second subject as the labels of the sample motion data.

In some embodiments, the processing device 140 may label the sample motion data using a labeling model to obtain the labels. The labeling model may be a trained machine learning model capable of recognizing motion errors in the sample image data and generating corresponding labels. The fourth acquisition module 720 may acquire sample image data corresponding to the sample motion data, and determine the labels of the sample motion data using the labeling model based on the correspondence between the sample image data and the sample motion data.

Operation 830, the motion evaluation model may be obtained by training an initial model based on the training sample set and the labels of the sets of sample motion data, the motion evaluation model being configured to evaluate motion data. In some embodiments, operation 830 may be executed by the training module 730.

In some embodiments, the processing device 140 may train the initial model based on the training sample set and the labels to obtain the motion evaluation model for evaluating motion data. The initial model may include, but is not limited to, a machine learning model. In some embodiments, the training process of the initial model based on the training sample set and the labels may include one or more iterations. In at least one current iteration, for each set of sample motion data, the processing device 140 may use the initial model to generate a predicted evaluation result. For example, the processing device 140 may input the sample motion data into the initial model to generate a predicted evaluation result corresponding to the sample motion data. The predicted evaluation result may include an error time and an error content. The error time may include an error start time and an error end time, and the error content may include an action type, a target part, an error type, etc., or any combination thereof. Furthermore, the processing device 140 may compare the predicted evaluation result with the labels to determine a loss function value. The loss function value may be used to measure a difference between the predicted evaluation result and the labels. Furthermore, the processing device 140 may determine whether a termination condition is met based on the loss function value. Exemplary termination conditions may include that the loss function value obtained in the current iteration is less than a preset threshold, a preset count of iterations are performed, the loss function value converges, etc., or any combination thereof. If the loss function value does not meet the termination condition, the iteration may continue to update the internal parameters of the initial model. If the loss function value meets the termination condition, the training process may be finished, and the initial model in the current iteration is taken as the motion evaluation model for evaluating motion data. In some embodiments, the processing device 140 may use the motion evaluation model to evaluate whether there are motion errors in the motion data in real time. In some embodiments, the processing device 140 may also provide feedback at an appropriate time (e.g., real-time, after the action ends, after the motion ends, etc.) when a motion error is identified, thereby helping the user to correct motion errors and improve the efficiency and safety of user motions, and ensuring scientific motion for the user.

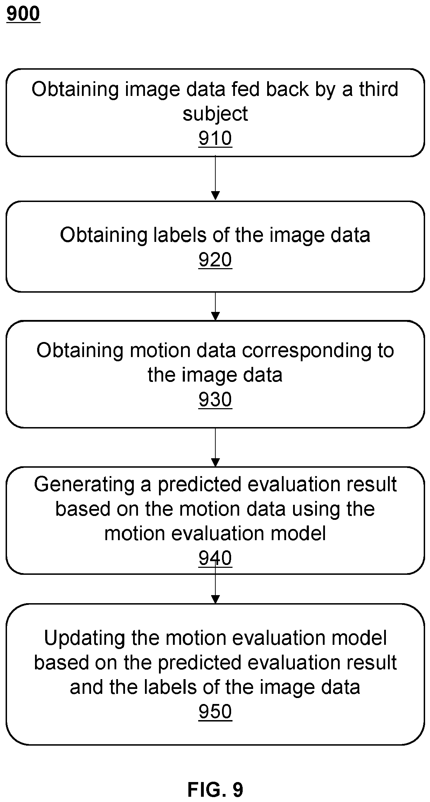

In some embodiments, the processing device 140 may update the motion evaluation model based on feedback data from a third subject. For example, the processing device 140 may obtain image data fed back by the third subject and obtain labels of the image data. Furthermore, the processing device 140 may obtain motion data corresponding to the image data. The motion data may include an electromyography signal, a posture signal, a mechanical signal, an electrocardiography signal, a respiratory signal, a sweat signal, etc., or any combination thereof. Furthermore, the processing device 140 may generate a predicted evaluation result based on the motion data using the motion evaluation model and update the motion evaluation model based on the predicted evaluation result and the labels of the image data. It should be noted that the first, second, and third subjects described in the present disclosure may be different subjects performing different operations, or may be the same object performing different operations. For example, the first subject may be a fitness trainee used to obtain sample motion data and sample image data, the second subject may be a fitness coach who labels the sample image data of the fitness trainee, and the third subject may be another user who provides feedback data. As another example, the first, second, and third subjects may all be the fitness coach. As yet another example, the third subject may be a different user from the first subject, which may increase the diversity of data used in generating and updating the motion evaluation model, thereby improving the accuracy of the motion evaluation model. More descriptions regarding updating the motion evaluation model based on feedback data may be found elsewhere in the present disclosure (e.g., FIG. 9 and relevant descriptions thereof), which is not repeated here.

In some embodiments, the processing device 140 may display the labels of the image data to the third subject through a terminal device. For example, the image data provided by the third subject (e.g., a fitness trainee) may be labeled by the second subject (e.g., a fitness coach) to determine the labels. The processing device 140 may obtain the labels from the second subject's terminal device to update the motion evaluation model. The processing device 140 may also send the labels or the image data with labels to the third subject. The third subject may view the labels on the terminal device.

In some embodiments, the processing device 140 may obtain motion data corresponding to the image data, such as an electromyography signal, a posture signal, a mechanical signal, an electrocardiography signal, a respiratory signal, a sweat signal, etc., or any combination thereof, and display the motion data to the third subject through a terminal device. For example, the processing device 140 may send the image data with labels and the corresponding motion data to the third subject, and display the image data with labels and the corresponding motion data for comparison. In some embodiments, to facilitate the third subject's intuitive viewing of the relationship between the labels of the image data and the corresponding motion data, statistical charts, curves, animated demonstrations, or other manners may be used to display the aforementioned content (e.g., the labels of the image data provided by the third subject or the image data with the labels, the motion data corresponding to the image data provided by the third subject, etc.) to the user. In some embodiments, when there is an error in the third subject's motion action, to facilitate the third subject to correct his/her motion error, the processing device 140 may also display reference image data and/or reference motion data corresponding to a correct action to the user. For example, the processing device 140 may display the reference image data and/or the reference motion data corresponding to the correct action together with the image data and/or the motion data corresponding to the third subject's incorrect action for comparison.

It should be noted that the above description regarding process 800 is merely for illustration and explanation, and does not limit the scope of application of the present disclosure. For those skilled in the art, various modifications and changes can be made to process 800 under the guidance of the present disclosure. However, these modifications and changes remain within the scope of the present disclosure.

FIG. 9 is a flowchart illustrating a process for updating a motion evaluation model according to some embodiments of the present disclosure. In some embodiments, process 900 may be executed through processing logic, which may include hardware (e.g., circuitry, dedicated logic, programmable logic, microcode, etc.), software (instructions running on processing devices to perform hardware simulation), etc., or any combination thereof. In some embodiments, one or more operations in process 900 may be implemented by the processing device 140 and/or the terminal device 150 shown in FIG. 1. In some embodiments, process 900 may be executed by the training module 730. In some embodiments, the process 900 may also be executed by a separate model training or updating device. The following descriptions take the execution of process 900 performed by the processing device 140 as an example.

According to FIG. 9, in some embodiments, process 900 may include the following operation.

Operation 910, image data fed back by the third subject may be obtained.

In some embodiments, the processing device 140 may obtain the image data fed back by the third subject. For example, the third subject may capture his/her fitness video through his/her terminal device and upload the fitness video. The processing device 140 may obtain the fitness video as the image data fed back by the third subject.

Operation 920, labels of the image data may be obtained.

In some embodiments, the processing device 140 may obtain the labels of the image data. For example, the third subject feeding back the image data may label the data before feedback to determine the labels of the image data. As another example, the processing device 140 may send the image data fed back by the third subject (e.g., a fitness trainee) to the second subject (e.g., a fitness coach), and the second subject may label the image data to determine the labels.

Operation 930, motion data corresponding to the image data may be obtained.

In some embodiments, the processing device 140 may obtain the motion data corresponding to the image data. The motion data may be used to represent the motion state of the third subject. For example, the processing device 140 may directly obtain the motion data corresponding to the image data from a motion data acquisition device (e.g., the motion data acquisition device 110). The motion data may be data collected during the same motion process as the image data.

Operation 940, a predicted evaluation result may be generated based on the motion data using the motion evaluation model.

In some embodiments, the processing device 140 may use the motion data as input to generate a predicted evaluation result using the motion evaluation model. In some embodiments, the predicted evaluation result may include an error time and an error content. The error time may include an error start time and an error end time, and the error content may include an action type, a target part, an error type, etc., or any combination thereof.

Operation 950, the motion evaluation model may be updated based on the predicted evaluation result and the labels of the image data.

In some embodiments, the processing device 140 may update the motion evaluation model based on the predicted evaluation result and the labels of the image data. For example, the processing device 140 may compare the predicted evaluation result with the labels of the image data to determine the difference between the predicted evaluation result with the labels. Furthermore, the processing device 140 may update the motion evaluation model based on the difference. For example, the processing device 140 may adjust the internal parameters of the motion evaluation model based on the difference.

In some embodiments, according to the method described in process 900, updating the motion evaluation model by obtaining feedback data from the third subject allows for efficient model updates without the need for extensive image data collection, which improves the efficiency of model updates. Additionally, obtaining a large amount of feedback data through the feedback of the third subject may improve the accuracy of the updated model.

It should be noted that the above description related to process 900 is merely for illustrative purposes and does not limit the scope of application of the present disclosure. Those skilled in the art can make various modifications and changes to process 900 under the guidance of the present disclosure. However, these modifications and changes remain within the scope of the present disclosure.

The beneficial benefits of the embodiments in the present disclosure may include, but are not limited to: (1) The method provided in the present disclosure for labeling motion data may significantly reduce the workload of labeling training sample data during the training process of the motion evaluation model, thereby shortening the training cycle and improving the training speed of the model; (2) Synchronizing image data with motion data based on the first time point corresponding to the marking action type in the image data and the second time point corresponding to the marking action type in the motion data ensures the consistency of the timeline between the image data and the motion data, thereby ensuring the accuracy of the labels obtained by labeling the motion data; (3) A motion evaluation model for evaluating motion data can be obtained based on the method for generating a motion evaluation model provided in the present disclosure such that the motion evaluation can be performed without collecting image data of the user in motion, thus improving the convenience and operability of motion evaluation; (4) Updating the motion evaluation model based on image data fed back by the user may further u the model, thereby improving the accuracy of the evaluation results.

It should be noted that different embodiments may yield different benefits. In various embodiments, the potential benefits may be any combination of the above or any other possible benefits.

The basic concepts have been described above, apparently, in detail, as will be described above, and does not constitute limitations of the disclosure. Although there is no clear explanation here, those skilled in the art may make various modifications, improvements, and modifications of present disclosure. This type of modification, improvement, and corrections are recommended in present disclosure, so the modification, improvement, and the amendment remain in the spirit and scope of the exemplary embodiment of the present disclosure.

At the same time, present disclosure uses specific words to describe the embodiments of the present disclosure. As "one embodiment," "an embodiment," and/or "some embodiments" means a certain feature, structure, or characteristic of at least one embodiment of the present disclosure. Therefore, it is emphasized and should be appreciated that two or more references to "an embodiment" or "one embodiment" or "an alternative embodiment" in various parts of present disclosure are not necessarily all referring to the same embodiment. Further, certain features, structures, or features of one or more embodiments of the present disclosure may be combined.

In addition, unless clearly stated in the claims, the order of processing elements and sequences, the use of numbers and letters, or the use of other names in the present disclosure are not used to limit the order of the procedures and methods of the present disclosure. Although the above disclosure discusses through various examples what is currently considered to be a variety of useful embodiments of the disclosure, it is to be understood that such detail is solely for that purpose, and that the appended claims are not limited to the disclosed embodiments, but, on the contrary, are intended to cover modifications and equivalent arrangements that are within the spirit and scope of the disclosed embodiments. For example, although the implementation of various components described above may be embodied in a hardware device, it may also be implemented as a software only solution, e.g., an installation on an existing server or mobile device.

Similarly, it should be appreciated that in the foregoing description of embodiments of the present disclosure, various features are sometimes grouped together in a single embodiment, figure, or description thereof for the purpose of streamlining the disclosure aiding in the understanding of one or more of the various embodiments. However, this disclosure does not mean that the present disclosure object requires more features than the features mentioned in the claims. Rather, claimed subject matter may lie in less than all features of a single foregoing disclosed embodiment.

In some embodiments, the numbers expressing quantities of ingredients, properties, and so forth, used to describe and claim certain embodiments of the application are to be understood as being modified in some instances by the term "about," "approximate," or "substantially". Unless otherwise stated, "about," "approximate," or "substantially" may indicate ±20% variation of the value it describes. Accordingly, in some embodiments, the numerical parameters used in the specification and claims are approximate values, and the approximation may change according to the characteristics required by the individual embodiments. In some embodiments, the numerical parameter should consider the prescribed effective digits and adopt a general digit retention method. Although in some embodiments, the numerical fields and parameters used to confirm the breadth of its range are approximate values, in specific embodiments, such numerical values are set as accurately as possible within the feasible range.

With respect to each patent, patent application, patent application disclosure, and other material cited in the present disclosure, such as articles, books, manuals, publications, documents, etc., the entire contents thereof are hereby incorporated by reference into the present disclosure. Application history documents that are inconsistent with the contents of the present disclosure or that create conflicts are excluded, as are documents (currently or hereafter appended to the present disclosure) that limit the broadest scope of the claims of the present disclosure. It should be noted that in the event of any inconsistency or conflict between the descriptions, definitions, and/or use of terms in the materials appended to the present disclosure and those described in the present disclosure, the descriptions, definitions, and/or use of terms in the present disclosure shall prevail.

At last, it should be understood that the embodiments described in the present disclosure are merely illustrative of the principles of the embodiments of the present disclosure. Other modifications that may be employed may be within the scope of the present disclosure. Thus, by way of example, but not of limitation, alternative configurations of the embodiments of the present disclosure may be utilized in accordance with the teachings herein. Accordingly, embodiments of the present disclosure are not limited to that precisely as shown and described.

## Claims

1. A method for labeling motion data, comprising:
obtaining motion data of a first subject when the first subject is in motion, the motion data representing a motion state of the first subject;
obtaining image data of the first subject when the first subject is in motion; and
obtaining labeled motion data by labeling the motion data based on the image data.

2. The method of claim 1, wherein the labeling the motion data based on the image data includes:
sending the image data to a second subject;
obtaining labels in labeled image data labeled by the second subject, the labels including a label time and a label content; and
labeling the motion data based on the labels.

3. The method of claim 2, wherein the labeling the motion data based on the labels includes:
synchronizing the motion data with the image data; and
labeling the motion data according to the labels based on the synchronized motion data and the synchronized image data.

4. The method of claim 3, wherein the synchronizing the motion data with the image data includes:
determining a marking action type;
determining a first time point corresponding to the marking action type in the image data;
determining a second time point corresponding to the marking action type in the motion data; and
synchronizing the motion data with the image data based on the first time point and the second time point.

5. The method of claim 2, wherein the label time includes an error start time and an error end time.

6. The method of claim 2, wherein the label content includes at least one of: an action type, a target part, or an error type.

7. The method of claim 6, wherein the error type includes at least one of: an injury error, a compensation error, an efficiency error, or a symmetry error.

8. The method of claim 3, further comprising:
modifying the labels based on the motion data.

9. The method of claim 1, wherein the image data includes at least one of: video data, 3D animation data, or model motion pictures of the first subject in motion.

10. A method for generating a motion evaluation model, comprising:
obtaining a training sample set including sets of sample motion data, each set of sample motion data representing a motion state of a first subject;
for each set of sample motion data, obtaining labels corresponding to the sample motion data, the labels including a label time and a label content corresponding to the sample motion data; and
obtaining the motion evaluation model by training an initial model based on the training sample set and the labels of the sets of sample motion data, the motion evaluation model being configured to evaluate motion data.

11. The method of claim 10, wherein for each set of sample motion data, the obtaining labels corresponding to the sample motion data includes:
obtaining sample image data corresponding to the sample motion data;
sending the sample image data to a second subject;
obtaining labels in labeled sample image data labeled by the second subject; and
determining the labels corresponding to the sample motion data based on the labels in the labeled sample image data.

12. The method of claim 11, wherein the determining the labels corresponding to the sample motion data based on the labels in the labeled sample image data includes:
synchronizing the sample motion data with the sample image data; and
determining the labels corresponding to the sample motion data based on the synchronized sample motion data and the synchronized sample image data.

13. The method of claim 12, wherein the synchronizing the sample motion data with the sample image data includes:
determining a marking action type;
determining a first time point corresponding to the marking action type in the sample image data;
determining a second time point corresponding to the marking action type in the sample motion data; and
synchronizing the sample motion data with the sample image data based on the first time point and the second time point.

14. The method of claim 11, wherein the sample image data includes at least one of: sample video data, sample 3D animation data, or sample model motion pictures.

15. The method of claim 10, wherein the label time includes an error start time and an error end time.

16. The method of claim 10, wherein the label content includes at least one of: an action type, a target part, or an error type.

17. The method of claim 16, wherein the error type includes at least one of: an injury error, a compensation error, an efficiency error, or a symmetry error.

18. The method of claim 10, further comprising:
modifying the labels based on the sample motion data.

19. The method of claim 10, wherein the obtaining labels corresponding to the sample motion data includes:
obtaining labeled sample motion data labeled by a second subject; and
determining the labels corresponding to the sample motion data based on the labeled motion data.

20. The method of claim 19, wherein the obtaining labels corresponding to the sample motion data further includes:
obtaining sample image data corresponding to the sample motion data; and
modifying the labels based on the sample image data.

21. The method of claim 10, wherein the obtaining labels corresponding to the sample motion data includes:
obtaining sample image data corresponding to the sample motion data; and
determining the labels corresponding to the sample motion data based on the sample image data using a labeling model.

22. The method of claim 10, wherein the training an initial model based on the training sample set and the labels of the sets of sample motion data includes one or more iterations, where a current iteration of the one or more iterations includes:
for each set of sample motion data, generating a predicted evaluation result using the initial model;
determining a loss function value by comparing the predicted evaluation results with the labels of the sample motion data;
determining whether the current iteration satisfies a termination condition based on the loss function value; and
in response to determining that the current iteration satisfies the termination condition, determining the initial model as the motion evaluation model.

23. The method of claim 10, further comprising:
updating the motion evaluation model based on feedback data of a third subject.

24. The method of claim 23, wherein the updating the motion evaluation model based on feedback data of a third subject includes:
obtaining image data fed back by the third subject;
obtaining labels corresponding to the image data fed back by the third subject;
obtaining motion data corresponding to the image data fed back by the third subject, the motion data representing a motion state of the third subject;
generating a predicted evaluation result based on the motion data using the motion evaluation model; and
updating the motion evaluation model based on the predicted evaluation result and the labels corresponding to the image data.

25. The method of claim 24, further comprising:
displaying the labels corresponding to the image data to the third subject.

26. The method of claim 25, further comprising:
displaying the motion data corresponding to the image data to the third subject.

27. A system for labeling motion data, comprising:
a first acquisition module, configured to obtain motion data of a first subject when the first subject is in motion, the motion data representing a motion state of the first subject;
a second acquisition module, configured to obtain image data of the first subject when the first subject is in motion; and
a labeling module, configured to label the motion data based on the image data.

28. A system for generating a motion evaluation model, comprising:
a third acquisition module, configured to obtain a training sample set including sets of sample motion data, each set of sample motion data representing a motion state of a first subject;
a fourth acquisition module, configured to for each set of sample motion data, obtain labels corresponding to the sample motion data, the labels including a label time and a label content corresponding to the sample motion data; and
a training module, configured to obtain the motion evaluation model by training an initial model based on the training sample set and the labels of the sets of sample motion data, the motion evaluation model being configured to evaluate motion data.

29. A computer-readable storage medium, comprising executable instructions that, when executed by at least one processor, direct the at least one processor to perform the methods of any one of claims 1 to 26.
